(19) Europäisches Patentamt / European Patent Office / Office européen des brevets

(11) **EP 3 318 646 B1**

(12) **EUROPEAN PATENT SPECIFICATION**

(45) Date of publication and mention
of the grant of the patent:
**20.02.2019 Bulletin 2019/08**

(51) Int Cl.:
**C12R 1/865** (2006.01)   **A21D 8/04** (2006.01)
**C12N 1/18** (2006.01)

(21) Application number: **16197393.8**

(22) Date of filing: **04.11.2016**

(54) **FREEZE-RESISTANT YEAST AND USES THEREOF**

FROSTBESTÄNDIGE HEFE UND VERWENDUNGEN DAVON

LEVURES RÉSISTANT AU FROID ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**09.05.2018 Bulletin 2018/19**

(83) **Declaration under Rule 32(1) EPC (expert solution)**

(73) Proprietor: **NextFerm Technologies Ltd.**
**2069208 Yokneam Illit (IL)**

(72) Inventors:
• **GENDELMAN, Moran**
**2018300 Misgav (IL)**
• **COHEN, Tzafra**
**3452718 Haifa (IL)**
• **MOR, Sivan**
**3951227 Tirat Carmel, P.O.10245 (IL)**
• **KHUTORIAN, Marina**
**3286201 Nesher (IL)**

(74) Representative: **Greiner, Elisabeth**
**df-mp Dörries Frank-Molnia & Pohlman**
**Patentanwälte Rechtsanwälte PartG mbB**
**Theatinerstraße 16**
**80333 München (DE)**

(56) References cited:
**US-A- 5 352 606**

• **A. TEUNISSEN ET AL: "Isolation and Characterization of a Freeze-Tolerant Diploid Derivative of an Industrial Baker's Yeast Strain and Its Use in Frozen Doughs", APPLIED AND ENVIRONMENTAL MICROBIOLOGY, vol. 68, no. 10, 1 October 2002 (2002-10-01), pages 4780-4787, XP055309217, US ISSN: 0099-2240, DOI: 10.1128/AEM.68.10.4780-4787.2002**
• **HINO ET AL: "New Freeze-tolerant yeast for frozen dough preparations", CEREAL CHEMISTRY, AACC INTERNATIONAL INC, US, vol. 64, no. 4, 1 January 1987 (1987-01-01), pages 269-275, XP002098766, ISSN: 0009-0352**
• **Yuji Oda ET AL: "Selection of Yeasts for Breadmaking by the Frozen-Dough Method", Applied and Environmental Microbiology, 1 October 1986 (1986-10-01), pages 941-943, XP055309321, United States Retrieved from the Internet: URL:https://www.ncbi.nlm.nih.gov/pmc/artic les/PMC239143/pdf/aem00133-0347.pdf**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to novel freeze-resistant baker's yeast (*Saccharomyces cerevisiae*) strains and uses thereof, for example for the preparation of fresh or frozen dough products, e.g., bread. It provides freeze-resistant baker's yeast strains, which are, e.g., obtainable from specific deposited strains, e.g., by breeding these strains with each other or with other *Saccharomyces cerevisiae* strains. The invention also relates to methods of using said strains or methods of preparing a dough or dough product or yeast product, as well as such products.

Both as convenience products for home use and as commercial half-finished products, frozen doughs or dough products, e.g., for bread, rolls or pastry, are becoming more and more important. Accordingly, freeze-resistance of baker's yeast is an increasingly important parameter. Classical yeast strains may be used for preparation of frozen dough, but typically, a two to threefold excess of yeast is used for preparation of such doughs, which may negatively affect the flavor of the baked product.

Furthermore, shelf-life of frozen yeast products is much longer than shelf-life of non-frozen, fresh or dried yeasts. For such applications, freeze-resistance of yeast also plays an important role.

Special freeze-resistant yeast strains are already available (e.g., US 5, 352, 606 B1, EP 1 209 225 A1, EP 1 541 671 A1), however, there is a need in the art for baker's yeast strains which are yet better adapted to freezing, e.g., which have a higher survival rate and/or better performance, such as increased fermentative ability after freezing.

This problem is solved by the present invention, in particular, by the subject matter of the claims.

The present invention provides a yeast strain OL-01 deposited as NCYC 4095, S3-02 deposited as NCYC 4094, FL-03 deposited as NCYC 4105, IS-310 deposited as NCYC 4106, CC-05 deposited as NCYC 4128 and KF-06 deposited as NCYC-4129. The yeast strain of the invention may be a baker's yeast strain.

Said yeast strain of the invention preferably is freeze-resistant and suitable for baking, e.g. for baking bread. The yeast strain of the invention may be haploid, diploid, triploid, tetraploid or aneuploid, it is, however, preferred that the yeast strain of the invention is tetraploid.

The baker's yeast strain of the invention is capable of surviving a regimen comprising freezing and thawing with a survival rate of at least 1 %, wherein the regimen may be as follows:

a) the yeast strain is grown on YPD medium for 15 h in a shaker-incubator at 30°C and 200rpm,

b) the yeast culture is harvested and re-suspended in YPD medium to a concentration of 5 $OD_{600}$,

c) 5 mL yeast suspensions in 50 mL tubes (typically, centrifuge tubes) are incubated at -20°C for 48 -72h,

d) followed by four freezing/thawing cycles, each freezing/thawing cycle comprising 1.5 h at 30°C and 200 rpm, and at least 1 h at -20°C,

wherein the survival rate is determined as the number of viable yeast colonies after said freeze/thawing cycles / the number of viable yeast colonies before said freeze/thawing cycles x 100%, and wherein the number of viable yeast colonies is measured using seeding decimal dilutions of yeast suspensions on YPD agar plates and incubation at 30°C for 48 hours.

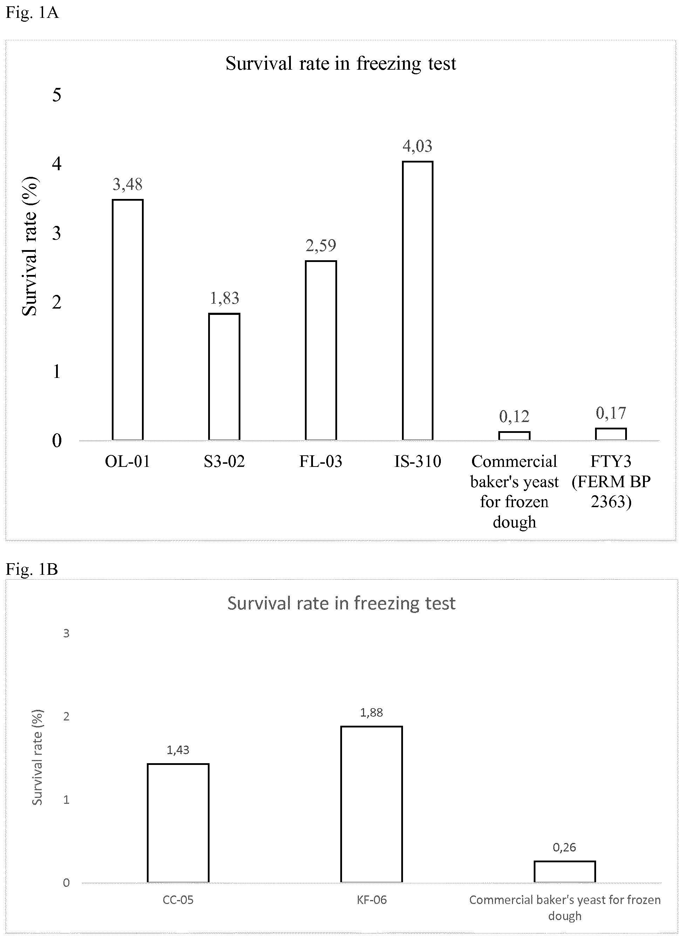

Yeast strains having a survival rate of at least 0.5% in said regimen are advantageous freeze-resistant strains of the invention. Preferably, the survival rate in said regimen is at least 1%, at least 1.5%, at least 1.75%, at least 2%, at least 2.5%, at least 3%, at least 3.5% or at least 4%., e.g., 0.5%-4.5%. The inventors showed that yeast stains of the invention have a survival rate of at least 1.2% or at least 1.5% in said regimen, whereas commercially available yeast for frozen dough and the freeze-resistant strain FTY3 (FERM BP 2363, cf. US 5,352,606 B1) only have a survival rate of 0.12 and 0.17%, respectively (Fig. 1).

Preferably, the yeast strains OL-01, S3-02, FL-03, IS-310, CC-05 and KF-06 have a survival rate in the described regimen of at least 0.5%, at least 1%, at least 1.75%, at least 2%, at least 2.5%, at least 3%, at least 3.5% or at least 4%., e.g., 0.5%-4.5%.

The fermentative performance of a yeast strain of the invention can advantageously be assessed in a liquid flour suspension (LFS), e.g., in the method as briefly described below, or in more detail in Example 5. This test advantageously assesses both freeze-resistance and fermentative performance.

Fermentative performance can, e.g., be tested by a method comprising propagating yeast cultures on 5mL YPD substrate for 15 h in a shaker-incubator at 30°C and at 200rpm, followed by freezing the cultures at -18°C for 3 days. One culture from each strain tested immediately for fermentative performance in a LFS e.g., non-sugar liquid flour suspension as described in Example 5. In brief, cultures were washed twice with distilled water and incubated with LFS for 1.5 hours. The LFS sample, after a certain time, forms two phases, a flour sediment at the bottom and a liquid phase on top. The other culture tested after freezing - is tested in the same manner. The yeast fermentative performance is graded after 90 min from 0 to 5 according to the following score:

*Time:* Yeast strains that initiate fermentation after 30 min of incubation receive 1 point, while yeast strains that start to ferment after 60 min receive 0.5 point and yeast stains with delayed initiation after 90 min do not receive any point.

*Bubbles:* Samples without appearance of bubbles in the liquid phase do not receive any points, samples with a small number of bubbles receive 0.5 point, while samples with a lot of bubbles receive 1 point.

*Holes:* Samples without holes in the solid phase do not receive points. Samples with small holes in it receive 0.5 point and dough with large holes receive 1 point. Samples with large connected holes receive 1.5 points.

*Foam:* Samples without foam on top of the liquid phase do not receive any points. Samples with a thin foam layer receive 0.5 point, samples with a thick foam layer receive 1 point, and samples with foam layer above 0.5cm receive 1.5 points.

Preferably, a yeast strain of the invention is assessed with a fermentative performance score of at least 1, at least 1.5, at least 2 or at least 3 after freezing. More preferably, the decrease in fermentative performance after freezing compared to the fermentative performance before freezing is at most 2 or at most 1.5 or at most 1.

The baker's yeast strain of the invention advantageously also has excellent fermentative ability. Fermentative ability may be determined following the protocol disclosed in Example 3, if non-frozen dough is used, or, if fermentative ability after freezing is of interest, in Example 4.

Preferably, fermentative ability of a yeast strain of the invention on non-frozen dough is at least comparable ($\pm$(up to -20 20%) to the fermentative ability of commercially available yeast strains such as FTY-3 (BP FERM 2363). A yeast strain of the invention may also have a superior ratio of fermentative ability of sugar dough (in particular, high sugar dough, e.g., 11% sugar) to non sugar dough. The inventors showed that in preferred yeast strains of the invention, e.g., OL-01, the fermentative ability on high sugar dough is exceptionally high, which is unique for yeast strains used in frozen dough application, preferably, the ratio of the fermentative ability on 11% sugar dough / fermentative ability on non sugar dough is at least 1.2, preferably, at least 1.3, at least 1.5, at least 1.7 or at least 1.9. OL-01 was shown to have a particularly high fermentative ability in high sugar dough.

With regard to fermentative ability in frozen dough, for example, the strain of the invention may show at least 90%, preferably, at least 91%, at least 93%, at least 95% fermentative ability after storage of dough prepared with said yeast strain at -20°C for 4 weeks and, preferably, after 8 weeks, compared to its fermentative ability after storage of said dough at - 20°C for 1 day.

Alternatively, or additionally, the strain may show at least 75%, preferably, at least 78% at least 80%, at least 82%, or at least 84% fermentative ability after storage of dough prepared with said yeast strain at -20°C for 4 weeks with at least 5, at least 10, at least 15, at least 20 or at least 25 freezing/thawing cycles performed during the first 2 weeks of storage (preferably at least 10 freezing/thawing cycles), wherein dough is transferred to 25°C (e.g., for 30 min) and returned to -20°C for (e.g., for at least 2 h) one freezing/thawing cycle, compared to its fermentative ability after storage of said dough at -20°C for 1 day.

The yeast strain of the invention may be capable of fermenting glucose and sucrose, as well as, optionally, maltose, galactose and raffinose. The yeast may be capable of assimilating carbon from glucose and sucrose, as well as, optionally, maltose, galactose and/or raffinose, which can be determined according to the method of Example 2. Typically, melibiose, and optionally, galactose and/or raffinose, cannot be assimilated. The strains of the invention each demonstrate a typical sugar spectrum to assimilate and grow on it.

The invention also provides a yeast product comprising yeast of a baker's yeast strain of the invention, wherein the yeast product is selected from the group comprising compressed yeast, crumbled yeast, cream yeast, active semi dry yeast, dry yeast, such as active dry yeast or instant active dry yeast, and frozen yeast. The yeast product may have a solids content of 10-99%.

Cream yeast, also designated liquid yeast, is used mainly by industrial bakeries. The main advantage of this product is that it is delivered directly in chilled containers for bakeries and can be pumped and dosed automatically. Cream yeast should be stored under cooled conditions, and its dry matter content is about 20% (T. Boekhout (Author, Editor), V. Robert (Editor), Yeasts in Food: Beneficial and Detrimental Aspects, Woodhead Publishing Series in Food Science, Technology and Nutrition, 2003, page 297).

Compressed yeast, also designated fresh compressed yeast is produced from the cream yeast. Filtration using a filter press or a rotary vacuum filter is typically used in order to eliminate part of the water contained in the cream yeast. The dry matter of this product varies between 28 and 35% , and depends on the country and the habits of the bakers. Compressed yeast with a lower dry matter content is darker in color and has a kneadable, rather plastic consistency. At high dry matter content the compressed yeast is more whitish and becomes crumbly. Fresh yeast must be preserved under cool conditions, e.g., at temperature of about 4°C or less, but it may also be frozen. If the cold chain is disturbed and the temperature is raised, the fresh yeast looses its activity rapidly. This product can be produced in different formats, such as small cubes for home baking, blocks of 500 grams or 1 kg and bags of 25 kg. The shelf life of the product is around 5 weeks. (T. Boekhout (Author, Editor), V. Robert (Editor), Yeasts in Food: Beneficial and Detrimental Aspects, Woodhead Publishing Series in Food Science, Technology and Nutrition, 2003, Page 297). Compressed yeast is a preferred yeast of the invention, in particular, for commercial use.

Crumbled yeast is a fresh yeast crumbled into irregular pieces of about 1 cm x 5-10 cm before being packed into 25-50 pound bags and stored under refrigeration to ensure stability during storage for about 4 weeks. Despite these precautionary measures, a loss of fermentation activity of 3-5% per week at 5-8°C is unavoidable, the packaged products may also be frozen. (Byong H. Lee, Fundamentals of Food Biotechnology, 2014, Wiley-Blackwell, page 184).

Active semi dry yeast has a solids content of 70-80 % (w/w). It is typically stored at 0-6°C, but it may also be frozen.

Active dry yeast products typically have a solids content of 90 or more % (w/w). All forms of active semi-dry or dry yeast can be stored at room temperature or refrigerated. Freezing is typically not required, even for long-term storage, but is possible. They are preferably packaged in vacuum or in an inert gas (like Nitrogen).

The yeast strains of the invention may also be used for the production of dry yeast products, i.e., active dry yeast (ADY), instant active dry yeast (Instant ADY) and protected active dry yeast (PADY). Compressed cake yeast may be treated with processing aids or antioxidants prior to drying, then extruded to spaghetti-like threads. After braking of the threads to 1.5-3 cm length, they are dried, e.g., in a tunnel on a conveyor belt type of dryer, e.g. fluid bed dryer. This kind of drying produces ADY. In order to activate ADY a separate step for rehydration with water is needed.

Instant ADYs with higher activity than ADYs require gentler drying, which is effected by fluidized-bed or airlift drying processes. The high activity of instant ADY is due to high porosity, there is no need for rehydration and can usually be added directly to dough. PADY contains an emulsifier and is dried by a special process to a moisture level of 5-6%. Use of antioxidants in this product reduces the adverse effect of oxygen and eliminates the need of special packaging. (Karel Kulp, Klaus Lorenz (ed.), Handbook of Dough Fermentations, 2003, CRC Press, ISBN 9780824742645).

The yeast product may be frozen yeast, e.g., frozen active semi dry yeast having 70-85%, in particular, 74-80% (w/w) dry matter, e.g., in the form of dry frozen "noodles". Frozen active semi dry yeast can be directly incorporated into flour without requiring prior thawing. The frozen yeast may also be a frozen active semi dry yeast product having diameter of less than 3mm and a dry matter content from 70 to 85% (w/w), preferably, 70-80% (w/w) dry matter, as disclosed in EP 1 209 225 A1 and CA 1299435. Frozen yeast can, e.g., be used for preparation of frozen dough. Frozen yeast products are stable for at least four weeks, preferably, at least 8 weeks, at least 3 months, at least 4 months, at least 6 months, at least 1 year, or at least 2 years.

The invention also provides a method for producing a yeast product, comprising culturing the baker's yeast strain of the invention, and, optionally, freezing the yeast.

Yeast products of the invention comprise yeast of a yeast strain of the invention. They may additionally comprise other baker's yeast, i.e., in the form of a mixture of different yeast strains.

The invention also provides a dough or dough product comprising the baker's yeast strain or the yeast product of the invention. The concentration of yeast (for compressed yeast of at least 30% dry matter) employed may be, e.g., 0.5% to 10% (w/w of flour), 1-7% or 2-5%. The concentration is typically comparable or less than concentrations of traditional yeast employed for comparable products. Preferably, in particular, if the yeast and/or the dough or dough product is frozen, a smaller concentration of yeast may be employed, preferably, about 30-90% of classical amounts recommended, 30-85%, 40-80%, 45-75% or 50-70%, without significantly affecting the texture of the obtained baked dough product.

A dough is obtainable by mixing yeast of the invention with at least one ingredient selected from the group comprising flour, and a liquid, with or without the addition of sugar. Typically, yeast is mixed with at least flour and a liquid. Typical ingredients used in dough, as well as suitable concentrations, are well known to the skilled person. The added liquid may be, e.g., water, milk, butter-milk, beer, optionally with the addition of oil. Water and milk powder may be used instead of milk.

Further optional dough ingredients are, e.g., sugar, eggs and/or fat. Egg powder may be used instead of fresh eggs. Fat may be, e.g., oil, butter or margarine. The dough may of course also comprise further dough ingredients, such as sourdough or a pre-dough mixture comprising at least one microorganism, spices (e.g., cinnamon, vanilla, pepper, garlic and/or herbs), grated lemon skin, baking improver, enzyme and/or salt. Fruits and/or vegetables, e.g., in dried or mashed form may be additional dough ingredients, e.g., raisins for sweet breads or cakes. In a preferred embodiment, e.g., for bread or rolls, the dough is sourdough.

The dough or dough product may comprise, e.g., 0-40% sugar, in particular, it may be non-sugar dough, lean sugar dough (1-10% sugar), or high sugar dough (more than 10% sugar, preferably, up to 25% or more). The inventors have been able to show that the yeast of the invention have comparable fermentative ability on non-sugar dough and lean sugar dough as commercially available yeast strains, and they, in particular, OL-01, have a particularly high fermentative ability on high sugar dough. The yeast strains of the invention are thus preferably used for high sugar dough, e.g., in particular for frozen high sugar dough. The dough optionally comprises up to 25% fat.

If a frozen yeast product of the invention is used, in particular if said yeast product has a small particle size, thawing is not required for preparation of dough, but the frozen product may be directly added to the dough ingredients, or to at least one other ingredient (e.g., flour). It is however of course possible to first thaw the frozen yeast product at a temperature of 0-37°C, e.g., at temperatures of about 0-8°C, or at room temperature (preferably, 20-25°C), or at 30-37°C, or with a stepwise increase in the temperatures, e.g., first bringing the temperature to about 0-8°C, and then to room temperature or higher temperatures.

Flour is a powder made by grinding uncooked cereal grains or other seeds or roots. Typically, flour is wheat flour, rye four, spelt flour, maize flour, oat flour, buckwheat flour, gluten-free flour or rice flour, or a mixture thereof, preferably, wheat flour. Flour types may be selected from the group comprising pastry flour, all purpose-flour, strong flour, very strong flour and/or whole-meal flour, or any of the types classified according to mineral content according to DIN 10355, e.g., type 405, type 550, type 650, type 812, type 1050 and/or type 1600. Flour may contain ascorbic acid. It is optionally treated with bleaching and/or maturing agents. If the dough contains sugar, in particular, if it is a high sugar dough, typically, pastry flour (corresponding to type 405) or type 550 flour from wheat is used.

The invention also provides frozen dough or frozen dough products. Frozen describes maintenance at -18°C or less or -20°C. Dough can be frozen by transferring the dough to a freezer of appropriate temperature, or, preferably, by using methods capable of lowering the temperature of dough more quickly, e.g., using a blast freezer.

In one embodiment, the frozen dough or dough product is obtained by mixing a frozen yeast product of the invention, optionally, after thawing, with at least one of the dough ingredients described above.

In another embodiment, the frozen dough or dough product is obtained by mixing a compressed yeast product of the invention with at least one of the dough ingredients described above.

In the context of the invention, a dough product is a formed dough piece. It may be raw, semi-raw (i.e., partly baked) or baked. It may also be frozen, in particular, a raw frozen dough piece. Semi-raw or baked dough pieces may also be frozen, but the yeast's freeze-resistant characteristics are most relevant where raw dough or dough products are frozen. In one embodiment, the dough product is a baked dough product obtained from a frozen dough or, preferably, a frozen dough product of the invention.

The dough or dough product of the invention may be bread dough, roll dough, pizza dough, cake dough, croissant dough, pretzel dough, bagel dough, all preferably already formed, bread, roll, pizza, croissant, pretzel, bagel and cake. Bread may be, e.g., baguette, ciabatta, whole meal bread, mixed bread, rye bread or sweet bread, e.g., yeast plait. Cakes include, e.g., pastries, muffins or cookies.

The invention further provides a method of preparing a dough or dough product of the invention, comprising steps of

   a) mixing the baker's yeast strain of the invention or the yeast product of the invention with at least one ingredient selected from the group comprising flour, and a liquid, such as water or milk, with or without the addition of sugar.

Further dough ingredients, e.g., as specified above, can be added, and a dough is obtained. The mixing step can be mixing of all dough ingredients together. Alternatively, it is step-wise mixing, wherein the yeast is first mixed with one of the ingredients, e.g., flour, or with a liquid and, wherein said liquid preferably has a temperature of 20-37°C when using active dry yeast and preferably 0-25°C when using compressed yeast to obtain a first mixture, and, e.g., after 0-30 min, this first mixture is mixed with the other ingredients. The mixing step typically includes kneading of the dough, e.g., for 1-15 min, preferably, 2-10 min. Kneading affects the consistence of dough by contribution to the development of a gluten network. Kneading is typically carried out at room temperature, but the temperature may also be controlled, e.g., at 16°C to 30°C and dough temperature may also be controlled, e.g., at 16-37°C. In particular, for preparation of frozen dough, the temperatures are below 25°C, e.g., at about 16-23°C.

The method of preparing a dough or dough product of the invention optionally comprises one or more further steps selected from the group comprising

   b) forming the dough:

   c) proofing the dough;

   d) freezing the dough;

   e) thawing the dough; and/or

   f) baking the dough.

Steps b), c) and d) may be carried out in any order, and repeated proofing steps may be used to achieve an optimal product. If step e) is carried out, it is carried out after step d). Step f) is typically carried out last, but it may be followed by a freezing and, optionally, thawing step.

In the context of the invention, proofing is considered the process wherein yeast converts fermentable sugars present in dough into carbon dioxide and ethanol. This increases the dough volume and affects rheology, e.g., the texture of the resulting product is lighter. Proofing is typically performed at a temperature between 20°C and 37°, preferably, at room temperature or at about 25°C. An increased humidity is beneficial, preferably, at least 75% or at least 85% relative humidity. The desired characteristics of the product, and accordingly, proofing conditions, depend on the type of product.

As the yeast of the invention has comparable or increased fermentative ability compared to commercially available yeasts, the conditions can be easily optimized by the skilled person.

Proofing the dough, e.g., for 15-90 min, is typically carried out after kneading. The dough may be formed before proofing. However, typically, after a proofing step, the dough is reworked, or punched, and, optionally formed. This may be followed by another proofing step, e.g., of 15-90 min.

A raw or frozen dough product may have undergone no proofing, full or partial proofing. Accordingly, freezing may be carried out after kneading, after a partial proofing period (typically, after forming), or after the full (maximum) required proofing. Preferably, freezing is performed after the full required proofing period. Throughout the invention, for convenience reasons, it is preferred that a formed dough product is frozen rather than the unformed dough.

In a preferred embodiment, for non-proofed frozen dough products, dough products are frozen after forming. After thawing, they can be proofed, followed by baking.

In another preferred embodiment, for proofed or at least partially proofed frozen dough products, dough products are formed, proofed (for about 60-100%, typically, about 70-80% of the typical full required proofing volume), and frozen. After thawing and, optionally, further proofing, they may be baked.

After freezing, the dough or dough product may be thawed before baking (at 0°C-37°C, preferably, at 20°C to 32°C), and, optionally, a proofing step may be performed. This may be particularly useful for heavy doughs such as dough containing sugar and/or fat. However, using the yeast of the invention, this is not required, i.e., the dough or dough product may be a frozen dough or dough product suitable for baking without thawing, e.g., a frozen dough in the shape of a baguette.

Advantageously, a frozen dough or dough product of the invention is stable for at least 2 weeks, at least 4 weeks, at least 8 weeks, at least 3 months, at least 6 months or at least a year when frozen at temperatures of -18°C. Stable in the context of the invention means that the product is still appropriate for human consumption, and maintains at least comparable characteristics as a corresponding fresh product.

At any time after forming, e.g., after forming, after proofing, after freezing, after thawing or after baking, additional finishing steps may be performed, e.g., adding toppings such as icing, glazing with egg yolk, milk, sugar, marmalade, or a mixture thereof, or fruit.

The dough product of the invention may also be a baked product. Baking may take place with or without steaming, e.g., at temperatures of about 160-220°C for times of 15-60 min. Conditions such as temperature and baking time depend on the product (e.g., dough type and size, desired crust) and can easily be determined by the skilled person. In the context of the invention, while baking typically takes place in an oven, baking is understood to include other ways of heating the dough product to temperatures inactivating the yeast, e.g., steaming, heating by microwave, frying, roasting and/or cooking.

The dough or dough product of the invention may also be packaged, e.g., in a can or a foil. Packaging may be under vacuum conditions or in modified atmosphere, preferably, with reduced oxygen content or in the absence of oxygen. The package may include instructions for producing a ready to eat dough product, e.g., for further steps of dough preparation and baking.

For example, a frozen dough product may obtainable by mixing the ingredients, kneading, proofing the dough (e.g., for 15-30 min at room temperature, preferably about 25°C, reworking and forming the dough, optionally, further proofing the dough at room temperature, preferably about 25°C, for 15-45 min, e.g., 30-40 min, and freezing (e.g., at -18° to -20°C). Before baking, the frozen dough product may be thawed (e.g., at 32°C, 75% relative humidity) for at least 5 or at least 10 min, such as for about 30-45 min. Preferably, thawing is sufficient to obtain a dough consistency which assures maximum volume of the product. The dough product may however also be directly baked out of the freezer, without thawing.

The invention also provides use of the baker's yeast strain of the invention or the yeast product of the invention for preparing a dough or dough product, wherein the dough or dough product may be a frozen dough or dough product.

The invention is further described in the following examples to illustrate the invention, but the examples are not intended to limit the scope of the invention. All references cited herein are herewith fully incorporated herein for all purposes.

**Fig. 1 Survival rate of yeast strains of the invention compared with state of the art commercial yeast strains.**

*Saccharomyces cerevisiae* strains OL-01, S3-02, FL-03, IS-310 **(A),** CC-05, KF-06 **(B),** commercial baker's yeast for frozen dough and freeze resistant strain FTY-3 (FERM BP 2363 (US 5,352,606 B1)) were grown on YPD medium, harvested and re-suspended in fresh YPD medium to a concentration of 5 $OD_{600}$. Yeast suspensions were incubated in -20°C for 48-72 hours, followed by four freezing/thawing cycles (1.5 h in 30°C/ at least 1 h in -20°C). Viable yeast counts were measured using seeding decimal dilutions of yeast suspensions on YPD agar plates and incubation in 30°C for 48 hours. Survival rate was determined as follows:

$$\text{Survival rate} = \text{Final number of yeast colonies} / \text{Initial number of yeast colonies} \times 100\%$$

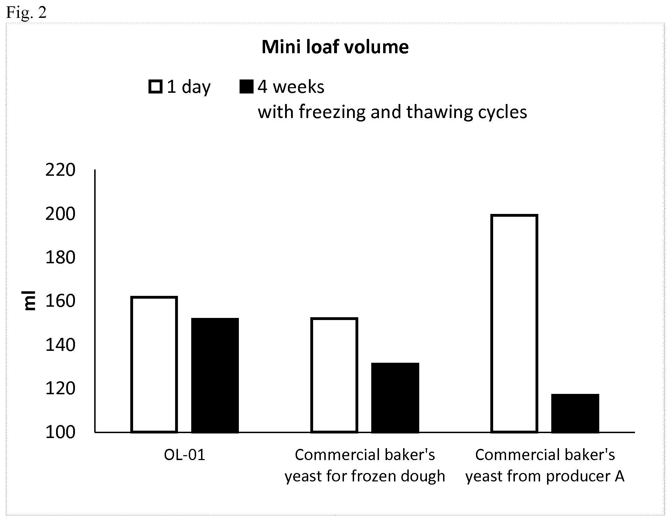

**Fig. 2 Mini loaf volume of frozen no sugar dough samples of yeast strain of the invention compared with state of the art commercial yeast strains**. *Saccharomyces cerevisiae* strain OL-01 culture obtained as described in Example 3, commercial baker's yeast for frozen dough and commercial baker's yeast for all purposes dough applications were used for preparation of non-sugar dough. Dough was prepared, divided to portions of 50 g, molded, and frozen to -20°C using a blast freezer, transferred to a polyethylene bag and then stored in a -20°C freezer for a period of up to 4 weeks. On one day storage at -20°C and 4 weeks storage at -20°C with freezing/thawing cycles after preparation, dough samples were thawed at 25°C for 20-30 min, proofed and baked in mini loaf pans. Mini loafs apparent volume was measured after cooling to room temperature according to a solids displacement method used to measure the volume of irregular solids.

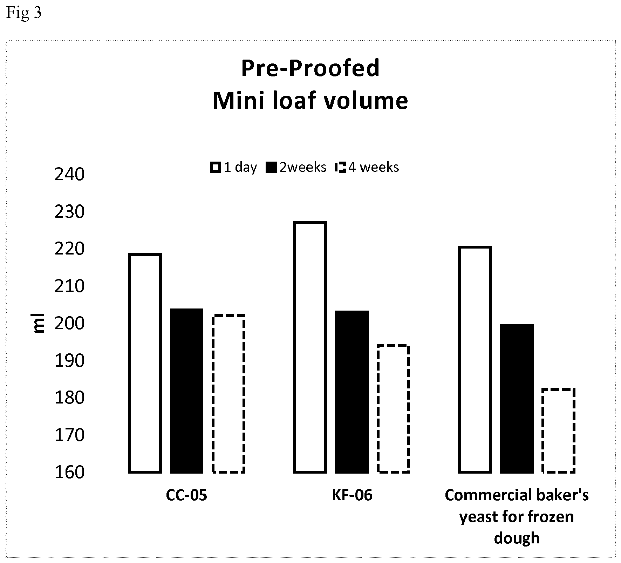

**Fig. 3 Pre-proofed mini loaf volume.** The Figure shows a comparison of the volume obtained with CC-05, KF-06 and commercial baker's yeast for frozen dough, as described in Example 7.2.

## Examples

## Example 1 New yeast strains and their morphology

New freeze-resistant *Saccharomyces cerevisiae* strains OL-01 (NCYC 4095), S3-02 (NCYC 4094), FL-03 (NCYC 4105), IS-310 (NCYC 4106), CC-05 (NCYC 4128) and KF-06 (NCYC 4129) were selected and deposited in NCYC under the Budapest Treaty. FL-03, IS-310 were obtained by breeding of offsprings of OL-1 and S3-02.

Each yeast strain was propagated in YPD medium (cf. Table 2) and observed under a microscope for morphological characteristics. Yeast colonies were grown on YPD-agar (cf. Table 2) and observed for their morphological properties.

Sporulation: yeast grown on YPD-agar plates was further seeded on SPO agar plates (cf. Table 2) and incubated at 25°C for 7 -10 days. Occurrence of sporulation was confirmed by microscope observation.

### Table 1: Morphological properties of the new yeast strains

| | OI-01 | S3-02 | FL-03 | IS-310 | CC-05 | KF-06 |
|---|---|---|---|---|---|---|
| Characteristic of the yeast cell | | | | | | |
| Shape | Round | Round | Round | Round | Round | Round |
| Size($\mu$m) | 4.4 | 5.3 | 5.7 | 5.2 | 5.2 | 5.0 |
| Sporulation | + | + | + | + | + | + |
| Budding | + | + | + | + | + | + |
| Film formation | - | - | - | - | - | - |
| Aggregation | - | + | + | + | + | + |
| Characteristic of yeast's colony | | | | | | |
| Form | Circular | Circular | Circular | Circular | Circular | Circular |
| Surface | Smooth | Smooth | Smooth | Smooth | Smooth | Smooth |
| Elevation | Raised | Raised | Raised | Raised | Raised | Raised |
| Margin | Entire | Entire | Entire | Entire | Entire | Entire |
| Size | Medium | Medium | Medium | Medium | Medium | Medium |
| Optic | Opaque | Opaque | Opaque | Opaque | Opaque | Opaque |

(continued)

| Characteristic of yeast's colony | | | | | | |
|---|---|---|---|---|---|---|
| Form | Circular | Circular | Circular | Circular | Circular | Circular |
| Color | White | White | White | White | White | White |
| Agar penetration | - | - | - | - | - | - |

**Table 2: Media composition**

| | YPD | YPD agar | SPO agar | YNB |
|---|---|---|---|---|
| Glucose (g) | 20 | 20 | 0.5 | - |
| Yeast extract (g) | 10 | 10 | 1 | - |
| Peptone (g) | 20 | 20 | | - |
| Agar (g) | - | 20 | 20 | 20 |
| $KH_2PO_4$ (g) | - | - | - | 4 |
| $MgSO_4*7H_2O$ (g) | - | - | - | 0.4 |
| $(NH4)_2SO_4$ (g) | - | - | - | 5 |
| Yeast nitrogen base (g) | - | - | - | 10 |
| $KCH_3COO$ (g) | - | - | 10 | - |
| Distilled water (mL) | 1000 | 1000 | 1000 | 1000 |

The freeze tolerance yeast strains can be obtained by conventional non GMO yeast improvement methods such as random mutagenesis, and breeding methods including: sexual recombination, and genome shuffling. These methods are detailed in two review articles: Giudici P. Solieri L. Pulvirenti A M.. Cassanelli S. 2005 Appl Microbiol Biotechnol 66: 622-628. Steensels J. Snoek T., Meersman E, Nicolino M . P., Voordeckers K. M & Verstrepen K. J. 2014 FEMS Microbiol Rev 38 947-995.

Said yeast can be obtained by random mutagenesis using mutagens (for instant chemical mutagen or UV) to generate genetic diversity in yeast population followed by selection toward a desired trait as described in Matsutani, K., Y. Fukuda, K. Murata, A. Kimura, I. Nakamura, and N. Yajima. 1990. J. Ferment. Bioeng.70:275-276. Alternatively, using sexual recombination that includes breeding of yeast strains usually involves one of the following approaches: single-spore culture grown after germination as described in Nakagawa, S. and Ouchi, K. 1994 Appl. Environ. Microbiol. 60, 3499-3502, and random garnets as described in Giudici P. Solieri L. Pulvirenti A M.. Cassanelli S. 2005 Appl Microbiol Biotechnol 66: 622-628.

Moreover, said yeast can be obtained using genome shuffling method, conducted by repetitive recombination between numerous parents followed by screening, as described in Paolo Giudici. Lisa Solieri. Andrea M. Pulvirenti. Stefano Cassanelli 2005 Appl Microbiol Biotechnol 66: 622-628.

A screening method for freeze-resistance can facilitate each of the breeding methods. It is possible to test the strains for freeze resistance by testing their viability after repeated freeze-thaw cycles (up to 200) in dough or after freezing in YPD media as described in Teunissen A, et al. 2002. Applied and Environmental Microbiology 68: 4780-4787 and in Tanghe A, et al., 2002. Applied and Environmental Microbiology 68 5981-5989.

## Example 2 - Carbon source assimilation

Carbohydrates assimilation was analyzed as follows. *Saccharomyces cerevisiae* strains OL-01, S3-02, FL-03, IS-310, CC-05 and KF-06 were grown on YPD medium for 15 hours with agitating (200 rpm), harvested and washed twice with sterilized water by centrifugation and re-suspended to a concentration of 0.5 OD/ml in YNB medium with various carbohydrates ( 2% w/v) respectively. The suspensions were incubated at 30°C with agitation of 200 rpm for 18 hours (OL-01, S3-02, FL-03, IS-310) or at 37°C with agitation of 100 rpm for 20 hours (CC-05, KF-06). Turbidity of the cultures was measured by absorbance at 600nm, to confirm yeast growth after 13 hours and after 18 hours. Estimation of growth ability was calculated in comparison to growth on glucose and without any sugar. Sugar types with which the yeast strain reached at least 80% of its concentration compared with growth on glucose was scored with "+". Sugar types with which the yeast strain reached more than 20% above its concentration compared with growth without any sugar and less than 80% of its concentration compared to growth on glucose scored "±". Sugar types with which the yeast strain concentration not exceed in more than 20% on its concentration without any sugar, scored "".

**Table 3 - Carbon source assimilation of new yeast strains**

|  | OL-01 | S3-02 | FL-03 | IS-310 | CC-05 | KF-06 |
|---|---|---|---|---|---|---|
| Glucose | + | + | + | + | + | + |
| Sucrose | + | + | + | + | + | + |
| Maltose | ± | + | + | + | + | ± |
| Galactose | ± | ± | ± | ± | + | + |
| Raffinose | ± | ± | ± | ± | NA | NA |
| Melibiose | - | - | - | - | - | - |

This experiment shows that yeast of invention demonstrates a typical carbon assimilation pattern.

**Example 3 - Fermentative ability of non-frozen dough**

3.1 Culturing the novel Saccharomyces cerevisiae strains.

A loopfull of fresh yeast was transferred from YPD agar plate to a 250 ml Erlenmeyer flask containing 60 mL molasses 12.5°Bx and incubated in a shaker incubator at 30°C with agitation of 200 rpm for 24 hours. Twenty mL of obtained yeast suspension were aseptically transferred into 1L Erlenmeyer flask containing 105 mL molasses 12.5°Bx and incubated under the same conditions for 48h, to produce seed yeast for main fermentation in a 5 L fermenter.

The culture solution was then inoculated into a 7 L fermenter vessel containing 2.5 L molasses medium (see Table 4) and grown under the conditions shown in Table 44 to obtain seed yeast for a 5 L fermenter culture.

The main culture medium shown in Table 4 was prepared in a volume of 5 L in a 7 L fermenter vessel and inoculated with the whole volume of the seed culture grown in a 2.5 L molasses medium in a 7 L fermenter, followed by culture under the following conditions.

**Table 4: Fermentation conditions**

| Ingredient | 2.5 L seed culture Batch | 5 L main culture Fed batch |
|---|---|---|
| Inoculum | 125 mL | 185 mL |
| Deionized water | 2125 mL | 3700 mL |
| Beat & Cane molasses ( optionally between 20 to 100% cane molasses) | 235 mL ~43°Bx | 818 mL~50 °Bx |
| Ammonia source | 12.5 mL $(NH_4)_2SO_4$ 20% | 107 mL Ammonia 11% |
| $H_3PO_4$ (85%) | 0.3 mL | 1.75 mL |
| $MgSO_4$ $7H_2O$ | 0.25 g | 0.5 g |
| Biotin | 1.5 mg | 3 mg |
| Thiamine | 6.25 mg | 12.5 mg |
| Pantothenic acid | 5 mg | 10 mg |
| **Cultured conditions** |  |  |
| Temperature | 30-32°C | 30-32°C |
| Aeration volume | 0.8 vvm | 1.6-2 vvm |
| Agitation | 200-600 rpm | 900-1000 rpm |
| pH | 5.0-5.5 | 5.0-5.5 |

(continued)

| Cultured conditions | | |
|---|---|---|
| Culturing time | 10 hr | 12 hr |

After completion of fermentation, yeast's cells were washed with sterile water and separated by centrifugation and filtration to obtain yeasts with 28-35% dry matter.

The fermentation procedure described above is described, e.g., in Yeast Strain Selection edited by Chandra J. Panchal, 1990, page 140, which is incorporated by reference, or in US4232045, US3617306A, EP 0 237 427 B2, the entire disclosures of which are also incorporated by reference.

3.2. Fermentative ability on non-frozen dough

3.2.1 Two types of dough samples were prepared according to the composition described in Table 5 using white wheat flour Type 550 (German specification, i.e., ash content 0.50-0.58%, extraction rate ~72%, gluten content of 9-11%).

Immediately after preparation, dough samples were analyzed for fermentative ability using the ANKOM[RF] Gas Production Measurement System (K. Müller-Auffermann et al, 2014. Brewing Science 67:72-80). The measurements were carried out in 30°C for 60 minutes. This system determines baker's yeast $CO_2$ production levels in terms of pressure and temperature converted to $CO_2$ volume. Table 6 shows the results obtained.

**Table 5: Dough composition**

| | Sugar [% w/w of flour] | Salt [% w/w of flour] | Yeast (30% solids) [% w/w of flour] | Fat [% w/w of flour] | Water [% w/w of flour] |
|---|---|---|---|---|---|
| Non sugar dough | 0 | 2 | 2 | 0 | 65 |
| 11% sugar dough | 11 | 2 | 6 | 15 | 60 |

**Table 6: Fermentative ability (ml $CO_2$) on non-frozen (fresh) doughs**

| | Non sugar dough [ml $CO_2$/hr/100gr dough] | 11% sugar dough [ml $CO_2$/hr/100gr dough] | Fermentative ability on 11% sugar dough / fermentative ability on non sugar dough |
|---|---|---|---|
| OL-01 | 93 | 176 | 1.9 |
| S3-02 | 106 | 124 | 1.2 |
| IS-310 | 96 | 127 | 1.3 |
| CC-05 | 96 | 161 | 1.7 |
| KF-06 | 86 | 137 | 1.6 |
| FTY-3 (BP FERM 2363) | 104 | 86 | 0.8 |
| Commercial baker's yeast use in frozen dough | 94 | 119 | 1.3 |
| Commercial baker's yeast from producer A | 127 | 161 | 1.3 |
| Commercial baker's yeast from producer B | 106 | 120 | 1.1 |
| Commercial baker's yeast from producer C | 110 | 125 | 1.1 |

3.2.2 Two types of dough samples were prepared according to the composition described in Table 7 using a bright white wheat flour, ash content ~0.4%, protein ~11%, wet gluten content of ~29%.

Immediately after preparation, dough samples were analyzed for fermentative ability using the ANKOM[RF] Gas Production Measurement System (K. Müller-Auffermann et al, 2014. Brewing Science 67:72-80). The measurements were carried out in 37°C for 60 minutes. This system determines baker's yeast $CO_2$ production levels in terms of pressure and temperature converted to $CO_2$ volume. Table 8 shows the results obtained.

**Table 7: Dough composition**

|  | Sugar [% w/w of flour] | Salt [% w/w of flour] | Yeast (30% solids) [% w/w of flour] | Water [% w/w of flour] |
|---|---|---|---|---|
| 2% sugar dough | 2 | 1.5 | 4 | 62 |
| 15 % sugar dough | 15 | 1.5 | 4 | 53 |

**Table 8: Fermentative ability (ml $CO_2$) on non-frozen (fresh) doughs**

|  | 2% sugar dough [ml $CO_2$] | 15% sugar dough [ml $CO_2$] |
|---|---|---|
| OL-01 | 300 | 182 |
| S3-02 | 350 | 168 |
| Commercial baker's yeast use in frozen dough | 310 | 148 |
| Commercial baker's yeast from producer A | 382 | 190 |
| Commercial baker's yeast from producer B | 318 | 134 |

Experiments 3.2.1 and 3.2.2 show that the tested novel strains show at least comparative fermentative ability to commercially available yeast strains on non-frozen dough. In preferred yeast strains of the invention, the fermentative ability on high sugar dough is exceptionally high, which is unique for yeast strains for application in frozen dough, preferably, the ratio of the fermentative ability on 11% sugar dough / fermentative ability on non sugar dough is at least 1.2, preferably, at least 1.3, at least 1.5, at least 1.7 or at least 1.9. OL-01 was shown to have a particularly high fermentative ability in high sugar dough.

**Example 4 - Fermentative ability on frozen dough**

4.1 *No sugar, frozen dough:*

4.1.1 Yeast cultures obtained in Example 3 were used for preparation of non-sugar dough containing about 2% (w/w of flour) salt, 4% (w/w of flour) yeast (30% solids), 53% (w/w of flour) of water using a bright white wheat flour. Dough was prepared, divided to portions of 50 g, molded, and frozen to -20°C using a blast freezer, transferred to a polyethylene bag and then stored in a -20°C freezer for a period of up to 4 weeks. One day and 4 weeks after preparation, dough samples were thawed at 25°C for 20-30 min and tested for $CO_2$ release during 90 min incubation at 37°C, using the ANKOM[RF] Gas Production Measurement System. This method determines baker's yeast $CO_2$ production levels in terms of pressure and temperature that are further converted to $CO_2$ volume. The measurements were carried out, and the total gas volume of 1 day and 4 weeks storage at -20°C samples was measured. To test the ability to withstand multiple freezing/thawing cycles, in a parallel experiment 10 cycles of thawing/freezing were performed during the first 2 weeks of storage, wherein doughs were transferred to 25°C for 30 min and returned to -20°C for at least two hours for one freezing/thawing cycle. Table 9 shows the results obtained.

**Table 9: Persistence of fermentative ability after 4 weeks of freezing presented as % from 1 day measurement**

|  | 4 weeks storage | 4 weeks storage with freezing/thawing cycles |
|---|---|---|
| OL-01 | 93% | 84% |
| S3-02 | 93% | 79% |

(continued)

| | 4 weeks storage | 4 weeks storage with freezing/thawing cycles |
|---|---|---|
| IS-310 | 91% | 77% |
| KF-06 | 97% | 86% |
| CC-05 | 96% | 88% |
| Commercial baker's yeast for frozen dough | 86% | 69% |
| Commercial baker's yeast from producer A | 79% | 47% |
| Commercial baker's yeast from producer B | 70% | 59% |

The experiment shows that the novel yeast strains of the invention may advantageously tolerate long term frozen storage, optionally with multiple freezing/thawing cycles better than commercially available yeast strains, e.g., showing at least 90%, preferably, at least 91% or at least 93% fermentative ability after 4 weeks storage at -20°C of the fermentative ability after 1 day of storage at -20°C, and at least 70%, at least 73%, preferably, at least 75%, at least 78% , at least 80%, at least 84% , at least 87% or at least 90% fermentative ability after 4 weeks storage at -20°C with freezing/thawing cycles of the fermentative ability after 1 day of storage at -20°C.

4.1.2 No sugar dough samples (50 g) obtained as in Example 4.1.1 and stored at -20°C for a period of up to 12 weeks. Dough samples were thawed at 25°C for 20-30 min and tested for $CO_2$ release during 60 min incubation at 37°C, using the ANKOM[RF] Gas Production Measurement System. The measurements were carried out, and the total gas volume produced after 1 day,after 8 weeks and after 12 weeks storage at -20°C was measured. Table 10 shows the results obtained.

**Table 10: Persistence of fermentative ability after 8 and 12 weeks of freezing presented as % from 1 day measurement**

| | 8 weeks storage | 12 weeks storage |
|---|---|---|
| OL-01 | 92% | 89% |
| KF-06 | 95% | XX |
| CC-05 | 95% | XX |
| Commercial baker's yeast use in frozen dough | 75% | 65% |
| Commercial baker's yeast from producer A | 59% | 31% |

The experiment shows that the novel yeast strains of the invention may advantageously tolerate long term frozen storage better than commercially available yeast strains, e.g., showing at least 80%, preferably, at least 82%, at least 83%, at least 85%, at least 87% or at least 90% fermentative ability after 8 and/or 12 weeks storage at -20°C of the fermentative ability after 1 day of storage at -20°C.

4.2 *No sugar, Pre-proofed frozen dough:*

Yeast cultures obtained in Example 3 were used for preparation of non-sugar dough containing about 1.5% (w/w of flour) salt, 2% (w/w of flour) yeast (30% solids), 65% (w/w of flour) of water using a bright white wheat flour.
Dough was prepared, divided to portions of 50 g, molded, proofed for 1 hr in a proofing chamber at 30°C and then frozen to -20°C using a blast freezer, transferred to a polyethylene bag and then stored in a -20°C freezer for a period of up to 4 weeks. One day, 2 weeks and 4 weeks after preparation, dough samples were thawed at 25°C for 20-30 min and tested for $CO_2$ release during 90 min incubation at 37°C, using the ANKOM[RF] Gas Production Measurement System. The measurements were carried out, and the total gas volume produced after 1 day, after 2 weeks and after 4 weeks storage at -20°C was measured. To test the ability to withstand multiple freezing/thawing cycles, in a parallel experiment 10 cycles of thawing/freezing were performed during the first 2 weeks of storage, wherein doughs were transferred to 25°C for 30 min and returned to -20°C for at least 2 h for one freezing/thawing cycle. Table 11A shows the results obtained.

**Table 11A: Persistence of fermentative ability after 2 and 4 weeks of Pre-proofed dough freezing presented as % from 1 day measurement using 2% yeast (30% solids)**

|  | 2 weeks storage | 2 weeks storage with freezing/thawing cycles | 4 weeks storage | 4 weeks storage with freezing/thawing cycles |
|---|---|---|---|---|
| OL-01 | 98% | 80% | 80% | 70% |
| FTY-3 (BP FERM 2363) | 92% | 55% | 65% | 44% |
| Commercial baker's yeast for frozen dough | 65% | 56% | 62% | 42% |

In an alternative setting, Yeast cultures obtained in Example 3 were used for preparation of non-sugar dough containing about 1% rapeseed oil (w/w of flour), 2.5% (w/w of flour) salt, 5% (w/w of flour) yeast (30% solids), 65% (w/w of flour) of water using a bright white wheat flour.

Dough was prepared, divided to portions of 80 g, molded, proofed for for 70 min at 20°C and then frozen to -20°C using a blast freezer transferred to a polyethylene bag and then stored in a -20°C freezer for a period of up to 2 weeks. One day and 2 weeks after preparation, dough samples were thawed at 25°C for 20-30 min and tested for $CO_2$ release during 90 min incubation at 37°C, using the ANKOM[RF] Gas Production Measurement System. The measurements were carried out, and the total gas volume produced after 1 day and after 4 weeks storage at -20°C was measured. To test the ability to withstand multiple freezing/thawing cycles, in a parallel experiment 10 cycles of thawing/freezing were performed during the first 2 weeks of storage, wherein doughs were transferred to 25°C for 30 min and returned to -20°C for at least 2 h for one freezing/thawing cycle. Table 11B shows the results obtained.

**Table 11B: Persistence of fermentative ability after four weeks of Pre-proofed dough freezing presented as % from 1 day measurement using 5% yeast (30% solids)**

|  | 4 weeks storage |
|---|---|
| KF-06 | 97% |
| CC-05 | 100% |
| Commercial baker's yeast for frozen dough | 69% |

The experiment shows that the novel yeast strains of the invention may advantageously tolerate long term frozen storage of pre-proofed frozen dough, optionally with multiple freezing/thawing cycles better than commercially available yeast strains, e.g., showing at least 94%, preferably, at least 95%, or, at least 98% fermentative ability after 2 weeks storage at - 20°C of the fermentative ability after 1 day of storage at -20°C, and at least 65%, preferably, at least 70% , or, at least 75% fermentative ability after 2 weeks storage at -20°C with freezing/thawing cycles of the fermentative ability after 1 day of storage at -20°C. Also, novel yeast strains preferably demonstrate at least 70%, preferable 80%, at least 90% or at least 95% fermentative ability after 4 weeks storage at -20°C of the fermentative ability after 1 day of storage at -20°C.

### 4.3 *1% sugar, Pre-proofed frozen dough:*

Yeast cultures obtained in Example 3 were used for preparation of lean sugar dough containing about 1% (w/w of flour) sugar, 2% (w/w of flour) salt, 5% (w/w of flour) yeast (30% solids), 1% (w/w of flour) rapeseed oil, 56% (w/w of flour) of water using a bright white wheat flour.

Dough was prepared, divided to portions of 70 g, molded, proofed for 40 minutes at room temperature, and then placed in -20°C freezer. Frozen pre-proofed dough samples were transferred to a polyethylene bag and then stored in a -20°C freezer for a period of up to 8 weeks. One day, 4 weeks and 8 weeks after preparation, dough samples were thawed at 32°C, about 75% relative humidity for 30-35 min and tested for $CO_2$ release during 90 min incubation at 37°C, using the ANKOM[RF] Gas Production Measurement System. The measurements were carried out, and the total gas volume produced after 1 day, after 4 weeks and after 8 weeks storage at -20°C was measured. To test the ability to withstand multiple freezing/thawing cycles, in a parallel experiment 10 cycles of thawing/freezing were performed during the first 2 weeks of storage, wherein doughs were transferred to 25°C for 30 min and returned to -20°C for at least 2 h

one freezing/thawing cycle. Table 12A shows the results obtained.

**Table 12A: Persistence of fermentative ability after 1 day, 4 weeks and 8 weeks of 1% sugar Pre-proofed dough freezing using 5% yeast (30% solids)**

| | 1 day storage | 4 weeks storage with freezing/thawing cycles | 8 weeks storage | 4 weeks storage with freezing/thawing cycles | 8 weeks storage |
|---|---|---|---|---|---|
| | [ml $CO_2$/hr/70gr dough] | | | % from 1 day storage | |
| OL-01 | 149 | 148 | 105 | 99% | 70% |
| IS-310 | 183 | 181 | 147 | 99% | 80% |
| Commercial baker's yeast from producer A | 210 | 121 | 93 | 57% | 44% |

In an alternative test, yeast cultures obtained in Example 3 were used for preparation of lean sugar dough containing about 1% (w/w of flour) sugar, 2% (w/w of flour) salt, 7% (w/w of flour) yeast (30% solids), 1% (w/w of flour) butter, 56% (w/w of flour) of water using a bright white wheat flour.

Dough was prepared, divided to portions of 70 g, molded, proofed in a proofing chamber for 10 min at 35°C and 10 min at room temperature, and then placed in a blast freezer. Frozen pre-proofed dough samples were transferred to a polyethylene bag and then stored in a -20°C freezer for a period of up to 4 weeks. One day and 4 weeks after preparation, dough samples were thawed at 32°C, about 75% relative humidity for 30-35 min and tested for $CO_2$ release during 90 min incubation at 37°C, using the ANKOM[RF] Gas Production Measurement System. The measurements were carried out, and the total gas volume produced after 1 day and after 4 weeks storage at -20°C was measured. To test the ability to withstand multiple freezing/thawing cycles, in a parallel experiment 10 cycles of thawing/freezing were performed during the first 2 weeks of storage, wherein doughs were transferred to 25°C for 30 min and returned to -20°C for at least 2 h one freezing/thawing cycle. Table 12B shows the results obtained.

**Table 12B: Persistence of fermentative ability after 1 day and 4 weeks of 1% sugar Pre-proofed dough freezing using 7% yeast (30% solids)**

| | 1 day storage | 4 weeks storage with freezing/thawing cycles | 4 weeks storage with freezing/thawing cycles |
|---|---|---|---|
| | [ml $CO_2$/hr/70gr dough] | | % from 1 day storage |
| KF-06 | 227 | 216 | 95 |
| Commercial baker's yeast for frozen dough | 227 | 148 | 65 |

The experiment shows that the novel yeast strains of the invention may advantageously tolerate long term frozen storage of 1% sugar pre-proofed frozen dough, optionally with multiple freezing/thawing cycles better than commercially available yeast strains, e.g., showing at least 80%, preferably, at least 90%, at least 95% fermentative ability after 4 weeks storage at -20°C with freezing/thawing cycles of the fermentative ability after 1 day of storage at -20°C.

### 4.4 *15% sugar, frozen dough:*

Yeast cultures obtained in Example 3 were used for preparation of high sugar dough containing about 15%(w/w of flour) sugar, 1.5% (w/w of flour) salt, 6% (w/w of flour) yeast (30% solids), 50% (w/w of flour) of water using a bright white wheat flour.

Dough was prepared, divided to portions of 50 g and molded, few fresh dough units were tested for $CO_2$ release, and the other units were frozen to -20°C using a blast freezer, transferred to a polyethylene bag and then stored in a -20°C freezer for a period of up to 12 weeks. One day,2, 8 and 12 weeks after preparation, dough samples were thawed at 25°C for 20-30 min and tested for $CO_2$ release during 90 min incubation at 37°C, using the ANKOM[RF] Gas Production Measurement System. The measurements were carried out, and the total gas volume of fresh dough, after 1 day, after 2, 8 and 12 weeks storage at -20°C was measured. To test the ability to withstand multiple freezing/thawing cycles, in a parallel experiment 10 cycles of thawing/freezing were performed during the first 2 weeks of storage, wherein doughs were transferred to 25°C for 30 min and returned to -20°C for at least 2 h for one freezing/thawing cycle. Table 13 shows the results obtained.

**Table 13: Persistence of fermentative ability after** 2, 8 and 12 **weeks of 15% sugar dough freezing**

| | Fresh dough | 2 weeks storage | 2 weeks storage with freezing/ thawing cycles | 8 weeks storage | 12 weeks storage |
|---|---|---|---|---|---|
| | ml $CO_2$/hr/100 gr | % from 1 day measurement | | | |
| OL-01 | 270 | 100% | 76% | 73% | 65% |
| IS-310 | 206 | 95% | 89% | 81% | 68% |
| FTY-3(BP FERM 2363) | 126 | 55% | 65% | 47% | 32% |

The experiment shows that the novel yeast strains of the invention may advantageously tolerate long term frozen storage of high sugar content (e.g., 15% sugar) frozen dough, optionally with multiple freezing/thawing cycles better than commercially available yeast strains, e.g., showing at least 80%, preferably, at least 90%, or at least 95% fermentative ability after 2 weeks storage at -20°C of the fermentative ability after 1 day of storage at - 20°C, at least 70%, preferably, at least 75% or at least 80% fermentative ability after 2 weeks storage at -20°C with freezing/thawing cycles of the fermentative ability after 1 day of storage at -20°C, or at least 65%, preferably, at least 70% or at least 75% fermentative ability after 8 weeks storage at -20°C with freezing/thawing cycles of the fermentative ability after 1 day of storage at -20°C or at least 60%, preferably at least 63% or at least 65% fermentative ability after 12 weeks storage at -20°C with freezing/thawing cycles of the fermentative ability after 1 day of storage at -20°C.

### Example 5 - Scoring of fermentative performance

Yeast cultures were propagated on 5mL YPD substrate for 15 h in a shaker-incubator at 30°C and at 200rpm, harvested and suspended in fresh YPD medium to the concentration of 5 $OD_{600}$. The 5 mL yeast suspensions in a 50 ml centrifuge tube (PP, Miniplast, Ein Shemer) were frozen at -18°C for 3 days, thawed for 20 min at room temperature and tested for their fermentative ability in a liquid flour suspension (LFS). The yeast samples were washed twice with distilled water, using a centrifugation at 4°C and 1300g for 8 min. The cultures were suspended in 10 mL lean (i.e. non-sugar) LFS (7.2g water, 0.23g salt, 2.57g white bright wheat flour) and incubated in a shaker-incubator at 37°C and at 100rpm. The LFS samples were (optionally) evaluated every 30 min. while a final grade was given after 90 min. The yeast fermentative performance was graded from 0 to 5 according to the following score: the grade was scored according to 4 parameters: time (1 point), bubbles loosed into a aqueous phase (1 point), holes inside the flour's sediment (1.5 points) and foam on the top of aqueous phase (1.5 point).

*Time:* Yeast strains that initiated fermentation after 30 min of incubation received 1 point, while yeast strains that started to ferment after 60 min received 0.5 point and yeast stains with delayed initiation after 90 min did not receive any point. Fermentation initiation was determinate by visual inspection of flour sediment and the presence of holes.

*Bubbles:* Samples without appearance of bubbles in the liquid phase did not receive any points, samples with a small number of bubbles (i.e., one bubble released into the liquid phase every 5-10 sec) received 0.5 point, while LFS with a lot of bubbles (at least one bubble released into the liquid phase every 2-5 sec) received 1 point.

*Holes:* LFS samples without holes in the solid phase did not receive points. LFS with small holes (below 1.5 mm in diameter) in it received 0.5 point, and LFS with large holes (1.5-2.5 mm in diameter) received 1 point. LFS with large connected holes (diameter greater than 5 mm) received 1.5 points.

*Foam:* LFS samples without foam on top of the liquid phase didn't receive any points. LFS with thin foam layer below 3 mm received 0.5 point, LFS with a thick layer of foam (3-5 mm) received 1 point and LFS with foam layer thicker than 5 mm received 1.5 points.

The results are shown in Table 14.

**Table 14: Fermentative performance score of new improved yeasts**

| Yeast strain | Score | |
|---|---|---|
| | Before freezing | After 3 days of freezing |
| **OL-01** | 3.5 | 1.5 |
| **S3-02** | 4 | 3 |
| **IS-310** | 2.5 | 2.5 |
| **CC-05** | 4.5 | 3.5 |
| **KF-06** | 4 | 2 |
| **Commercial baker's yeast for frozen dough** | 3.5 | 0 |

**Example 6 - Yeast survival test**

*Saccharomyces cerevisiae* strains OL-01, S3-02, FL-03, IS-310, CC-05, KF-06, commercial baker's yeast for frozen dough and freeze resistant strain FTY-3 (FERM BP 2363 (US 5,352,606 B1)) were grown on YPD medium, harvested and re-suspended in fresh YPD medium to a concentration of 5 $OD_{600}$. A sample was taken from each yeast suspension and immediately seeded on YPD agar plates to determinate the initial number of colonies. 5 mL yeast suspensions in a 50 ml centrifuge tube (PP, e.g., from Miniplast, Ein Shemer) were incubated in -20°C for 48-72 hours, followed by four freezing/thawing cycles (1.5 h in 30°C/ at least 1 h in -20°C). Viable yeast counts were measured using seeding decimal dilutions of yeast suspensions on YPD agar plates and incubation in 30°C for 48 hours.
Survival rate was determined as follows:

$$\text{Survival rate} = \text{Final number of yeast colonies / Initial number of yeast colonies x } 100\%$$

The results are shown in Fig. 1A and B.

**Example 7 - Baking test of frozen dough**

7.1. *Mini loaf volume:*

Non-sugar dough samples (50 g) obtained as in Example 4.1.1 after 1 day storage at -20°C and after 4 weeks storage at -20°C with freezing and thawing cycles (as described in Example 4.1) were thawed at 25°C for 20-30 min and proofed in a proofing chamber with above 85% relative humidity at 35°C for 70 min, and then baked in mini loaf pans at 195°C for 7-8 min. Mini loafs apparent. volume was measured after cooling to room temperature according to a solids displacement method used to measure the volume of irregular solids (Physical Properties of Foods, Serpil Sahin, Servet Gülüm Sumnu, page 20) using poppy seeds. The results are shown in Fig. 2.

7.2. *Pre-Proofed mini loaf volume:*

Non-sugar dough samples (50-80g) obtained as in Example 4.2 after 1 day storage at -20°C and after 4 weeks storage at -20°C as frozen pre-proofed mini loafs were directly baked, without thawing stage, according to the following baking program:

| Temp. | Steam | Time |
|---|---|---|
| 90°C | + | 4min |
| 110°C | + | 6min |
| 190°C | - | 11min |
| 185°C | - | 4min |

The mini loafs' apparent volume was measured after cooling to room temperature according to a solids displacement method used to measure the volume of irregular solids (Physical Properties of Foods, Serpil Sahin, Servet Gülüm Sumnu, page 20) using poppy seeds. The results are shown in Fig. 3.

7.3. *Mini loaf proofing time:*

Non-sugar dough samples (50 g) obtained as in Example 4.1 after 4 weeks storage at -20°C, optionally, with freezing and thawing cycles (as described in Example 4.1), were thawed at 25°C for 20-30 min and proofed in a proofing chamber at 35°C until they reached a predetermined height of 3.5 cm in a clear plastic box having the dimensions 5.8x8.6x4.5WxLxH [cm$^3$] maximally, for 120 min. The results are shown in Table 155.

**Table 15: Proofing time after 4 weeks freezing and 4 weeks freezing with freezing and thawing cycles**

|  | 4 weeks storage | 4 weeks with freezing and thawing cycles |
| --- | --- | --- |
| OL-01 | 65 min | 102 min |
| KF-06 | 85 min | 103 min |
| CC-05 | 85 min | 98 min |
| Commercial baker's yeast for frozen dough | 95 min | 120 min |
| Commercial baker's yeast from producer A | 90 min | >120 min |

The baking data above strengthens the data presented in previous examples that the novel yeast strains of the invention may advantageously tolerate long term frozen storage, optionally with multiple freezing/thawing cycles better than commercially available yeast strains, e.g., showing the smallest rate of mini loaf volume decrease after 4 weeks storage at -20°C with freezing/thawing cycles and the shortest proofing time after 4 weeks storage at -20°C and after 4 weeks storage at -20°C with freezing/thawing cycles in comparison to the commercial yeast strains presented above.

**Claims**

1.  A baker's yeast strain, wherein the strain is OL-01 deposited as NCYC 4095, S3-02 deposited as NCYC 4094, FL-03 deposited as NCYC 4105, IS-310 deposited as NCYC 4106, CC-05 deposited as NCYC 4128 or KF-06 deposited as NCYC 4129.

2.  The baker's yeast strain of claim 1, wherein the strain is CC-05 deposited as NCYC 4128.

3.  The baker's yeast strain of claim 1, wherein the strain is KF-06 deposited as NCYC 4129.

4.  A yeast product comprising yeast of a baker's yeast strain of any of claims 1 to 3, wherein the yeast product is selected from the group comprising cream yeast, compressed yeast, crumbled yeast, semi active dry yeast, active dry yeast and frozen yeast.

5.  A dough or dough product comprising the baker's yeast strain or the yeast product of any of the preceding claims.

6.  The dough or dough product of claim 5, which is frozen.

7.  The dough product of claim 5, wherein the dough product is a baked dough product obtained from a frozen dough product of claim 6.

8.  The dough or dough product of any of claims 5 to 7, wherein the dough or dough product is selected from the group comprising bread dough, roll dough, pizza dough, pretzel dough, bagel dough, cake dough, croissant dough, bread, roll, pizza, croissant, pretzel, bagel and cake.

9.  The dough or dough product of any of claims 5 to 8, wherein the dough or dough product is selected from the group comprising non-sugar dough, lean sugar dough (1-10% sugar), and high sugar dough (more than 10% sugar), wherein the dough preferably is a high sugar dough.

10. A method of preparing a dough or dough product of any of claims 5 to 9, comprising steps of

a) mixing the baker's yeast strain of any of claims 1 to 3 or the yeast product of claim 4 with at least one ingredient selected from the group comprising flour and a liquid,

the method optionally comprising one or more further steps selected from the group comprising

b) forming the dough;
c) proofing the dough;
d) freezing the dough;
e) thawing the dough; and/or
f) baking the dough.

11. Use of the baker's yeast strain of any of claims 1 to 3, or the yeast product of claim 4 for preparing a dough or dough product, wherein the dough is optionally frozen.

12. A method for producing a yeast product comprising culturing the baker's yeast strain of any of claims 1 to 3, and, optionally, freezing the yeast.

**Patentansprüche**

1. Backhefestamm, wobei der Stamm OL-01, hinterlegt als NCYC 4095, S3-02, hinterlegt als NCYC 4094, FL-03, hinterlegt als NCYC 4105, IS-310, hinterlegt als NCYC 4106, CC-05, hinterlegt als NCYC 4128 oder KF-06, hinterlegt als NCYC 4129, ist.

2. Backhefestamm nach Anspruch 1, wobei der Stamm CC-05, hinterlegt als NCYC 4128 ist.

3. Backhefestamm nach Anspruch 1, wobei der Stamm KF06, hinterlegt als NCYC 4129, ist.

4. Hefeprodukt, das Hefe eines Backhefestamms nach einem der Ansprüche 1 bis 3 umfasst, wobei das Hefeprodukt ausgewählt ist aus der Gruppe umfassend Cremehefe, komprimierte Hefe, zerbröckelte Hefe, halbaktive Trockenhefe, aktive Trockenhefe und gefrorene Hefe.

5. Teig- oder Teigprodukt, das den Backhefestamm oder das Hefeprodukt nach einem der vorstehenden Ansprüche umfasst.

6. Teig- oder Teigprodukt nach Anspruch 5, das gefroren ist.

7. Teigprodukt nach Anspruch 5, wobei das Teigprodukt ein gebackenes Teigprodukt ist, das aus einem gefrorenen Teigprodukt nach Anspruch 6 erhalten wird.

8. Teig oder Teigprodukt nach einem der Ansprüche 5 bis 7, wobei der Teig oder das Teigprodukt ausgewählt ist aus der Gruppe umfassend Brotteig, Walzenteig, Pizzateig, Brezelteig, Bagelteig, Kuchenteig, Croissantteig, Brot, Rolle, Pizza, Croissant, Brezel, Bagel und Kuchen.

9. Teig- oder Teigprodukt nach einem der Ansprüche 5 bis 8, wobei der Teig oder das Teigprodukt ausgewählt ist aus der Gruppe umfassend ungezuckerten Teig, Magerzuckerteig (1-10 % Zucker) und stark gezuckerten Teig (mehr als 10 % Zucker), wobei der Teig vorzugsweise ein stark gezuckerter Teig ist.

10. Verfahren zum Herstellen eines Teiges oder Teigproduktes nach einem der Ansprüche 5 bis 9, das die folgenden Schritte umfasst:

a) Mischen des Backhefestamms nach einem der Ansprüche 1 bis 3 oder des Hefeprodukts nach Anspruch 4 mit mindestens einem Inhaltsstoff, der ausgewählt ist aus der Gruppe umfassend Mehl und eine Flüssigkeit,

wobei das Verfahren wahlweise einen oder mehrere weitere Schritte umfasst, die ausgewählt sind aus der Gruppe umfassend

b) Bilden des Teigs,
c) Gehenlassen des Teigs,
d) Einfrieren des Teigs,
e) Auftauen des Teigs, und/oder
f) Backen des Teigs.

11. Verwendung des Backhefestamms nach einem der Ansprüche 1 bis 3 oder des Hefeprodukts nach Anspruch 4 zur Herstellung eines Teiges oder Teigprodukts, wobei der Teig wahlweise eingefroren ist.

12. Verfahren zur Herstellung eines Hefeprodukts, das das Kultivieren des Backhefestamms nach einem der Ansprüche 1 bis 3, und wahlweise das Einfrieren der Hefe umfasst.

**Revendications**

1. Souche de levure de boulanger, dans laquelle la souche est OL-01 déposée en tant que NCYC 4095, S3-02 déposée en tant que NCYC 4094, FL-03 déposée en tant que NCYC 4105, IS-310 déposée en tant que NCYC 4106, CC-05 déposée en tant que NCYC 4128 ou KF-06 déposée en tant que NCYC 4129.

2. Souche de levure de boulanger selon la revendication 1, dans laquelle la souche est CC-05 déposée en tant que NCYC 4128.

3. Souche de levure de boulanger selon la revendication 1, dans laquelle la souche est KF-06 déposée en tant que NCYC 4129.

4. Produit de levure comprenant de la levure d'une souche de levure de boulanger selon l'une quelconque des revendications 1 à 3, dans lequel le produit de levure est choisi dans le groupe comprenant une levure crème, une levure comprimée, une levure émiettée, une levure sèche semi-active, une levure sèche active et une levure congelée.

5. Pâte ou produit de pâte comprenant la souche de levure de boulanger ou le produit de levure selon l'une quelconque des revendications précédentes.

6. Pâte ou produit de pâte selon la revendication 5, qui est congelé.

7. Produit de pâte selon la revendication 5, dans lequel le produit de pâte est un produit de pâte cuit obtenu à partir d'un produit de pâte congelé selon la revendication 6.

8. Pâte ou produit de pâte selon l'une quelconque des revendications 5 à 7, dans lequel la pâte ou le produit de pâte est choisi dans le groupe comprenant une pâte à pain, une pâte à roulé, une pâte à pizza, une pâte à bretzel, une pâte à bagel, une pâte à gâteau, une pâte à croissant, un pain, un roulé, une pizza, un croissant, un bretzel, un bagel et un gâteau.

9. Pâte ou produit de pâte selon l'une quelconque des revendications 5 à 8, dans lequel la pâte ou le produit de pâte est choisi dans le groupe comprenant une pâte non sucrée, une pâte pauvre en sucre (1 à 10 % de sucre) et une pâte à haute teneur en sucre (plus de 10 % de sucre), dans lequel la pâte est de préférence une pâte à haute teneur en sucre.

10. Procédé de préparation d'une pâte ou d'un produit de pâte selon l'une quelconque des revendications 5 à 9, comprenant des étapes consistant à

a) mélanger la souche de levure de boulanger selon l'une quelconque des revendications 1 à 3 ou le produit de levure selon la revendication 4 avec au moins un ingrédient choisi dans le groupe comprenant de la farine et un liquide,

le procédé comprenant éventuellement une ou plusieurs étapes supplémentaires choisies dans le groupe comprenant

b) le formage de la pâte ;

    c) la fermentation de la pâte ;
    d) la congélation de la pâte ;
    e) la décongélation de la pâte ; et/ou
    f) la cuisson de la pâte.

11. Utilisation de la souche de levure de boulanger selon l'une quelconque des revendications 1 à 3, ou du produit de levure selon la revendication 4 pour la préparation d'une pâte ou d'un produit de pâte, dans lequel la pâte est éventuellement congelée.

12. Procédé de production d'un produit de levure comprenant la culture de la souche de levure de boulanger selon l'une quelconque des revendications 1 à 3 et, éventuellement, la congélation de la levure.

Fig. 1A

Fig. 1B

Fig. 2

**Mini loaf volume**

□ 1 day    ■ 4 weeks
with freezing and thawing cycles

ml

220
200
180
160
140
120
100

OL-01 — Commercial baker's yeast for frozen dough — Commercial baker's yeast from producer A

Fig 3

Pre-Proofed
Mini loaf volume

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5352606 B1
- EP 1209225 A1
- EP 1541671 A1
- CA 1299435

- US 4232045 A
- US 3617306 A
- EP 0237427 B2

**Non-patent literature cited in the description**

- Yeasts in Food: Beneficial and Detrimental Aspects. Woodhead Publishing Series in Food Science, Technology and Nutrition. 2003, 297
- **BYONG H. LEE.** Fundamentals of Food Biotechnology. Wiley-Blackwell, 2014, 184
- Handbook of Dough Fermentations. CRC Press, 2003
- **GIUDICI P. ; SOLIERI L. ; PULVIRENTI A M. ; CASSANELLI S.** *Appl Microbiol Biotechnol,* 2005, vol. 66, 622-628
- **STEENSELS J. SNOEK T. ; MEERSMAN E, NICOLINO M . P. ; VOORDECKERS K. M ; VERSTREPEN K. J.** *FEMS Microbiol Rev,* 2014, vol. 38, 947-995

- **MATSUTANI, K. ; Y. FUKUDA ; K. MURATA ; A. KIMURA ; I. NAKAMURA ; N. YAJIMA.** *J. Ferment. Bioeng.,* 1990, vol. 70, 275-276
- **NAKAGAWA, S. ; OUCHI, K.** *Appl. Environ. Microbiol.,* 1994, vol. 60, 3499-3502
- **PAOLO GIUDICI. ; LISA SOLIERI. ; ANDREA M. ; PULVIRENTI. ; STEFANO CASSANELLI.** *Appl Microbiol Biotechnol,* 2005, vol. 66, 622-628
- **TEUNISSEN A et al.** *Applied and Environmental Microbiology,* 2002, vol. 68, 4780-4787
- **TANGHE A et al.** *Applied and Environmental Microbiology,* 2002, vol. 68, 5981-5989
- Yeast Strain Selection. 1990, 140
- **K. MÜLLER-AUFFERMANN et al.** *Brewing Science,* 2014, vol. 67, 72-80